(19) Europäisches Patentamt
European Patent Office
Office européen des brevets

(11) Publication number: **0 350 080**
**A2**

# (12) EUROPEAN PATENT APPLICATION

(21) Application number: 89115176.3

(51) Int. Cl.⁴: A61K 31/135

(22) Date of filing: 23.04.82

(30) Priority: 24.04.81 US 257453
14.05.81 US 263656
25.06.81 US 277378

(43) Date of publication of application:
10.01.90 Bulletin 90/02

(60) Publication number of the earlier application in accordance with Art.76 EPC: 0 064 646

(84) Designated Contracting States:
AT BE CH DE FR GB IT LI LU NL SE

(71) Applicant: E.R. Squibb & Sons, Inc.
Lawrenceville-Princeton Road
Princeton, N.J. 08540(US)

(72) Inventor: Sudilovsky, Abraham
712 Winchester Avenue
Lawrenceville, N.J.(US)
Inventor: Muir, John Gordon
R.R.1, Box 342 Poor Farm Road
Pennington, N.J.(US)

(74) Representative: Vossius & Partner
Siebertstrasse 4 P.O. Box 86 07 67
D-8000 München 86(DE)

## (54) Use of nadolol for inhibiting the onset of migraine headaches.

(57) Nadolol compositions are provided for treating glaucoma and for inhibiting the onset of migraine headaches.

EP 0 350 080 A2

## USE OF NADOLOL FOR INHIBITING THE ONSET OF MIGRAINE HEADACHES

It has been discovered that nadolol or an ester thereof can be used for preventing or inhibiting onset of migraine headaches in mammalian species wherein a therapeutically effective amount of nadolol or an ester thereof is systemically, e.g. orally or parenterally, administered.

The term "nadolol" as employed herein refers to the beta blocker 2,3-cis-1,2,3,4-tetrahydro-5-[2-hydroxy-3-(tert-butylamino)propoxy]-2,3-naphthalenediol (and pharmaceutically acceptable acid-addition salts thereof)

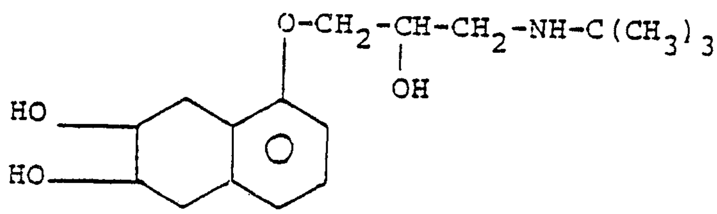

which is disclosed in U.S. Patent Nos. 3,935,267 and 3,982,021.

Esters of nadolol included herein are the mono-, di- and tri-esters (and pharmaceutically acceptable acid-addition salts thereof).

The di-esters have the structure

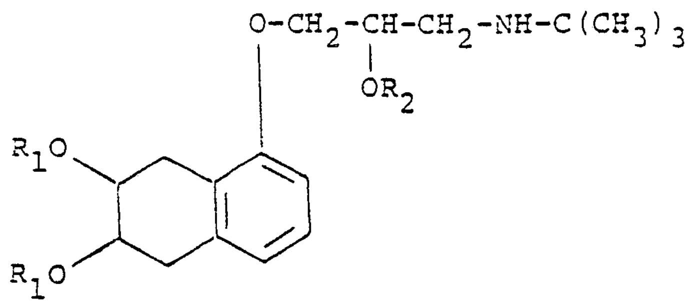

wherein both $R_1$ groups are acyl, preferably acetyl, and $R_2$ is hydrogen.

Esters of nadolol wherein both $R_1$ groups are hydrogen and $R_2$ is acyl (mono-ester), preferably acetyl, and wherein both $R_1$ groups are acyl, preferably acetyl, and $R_2$ is acyl, preferably acetyl (tri-esters) are disclosed in British Patent Specification No. 1,559,987.

In carrying out the method for preventing or inhibiting the onset of migraine headaches, the nadolol or nadolol ester or a physiologically acceptable acid-addition salt may be administered to mammalian species, such as monkeys, dogs, cats, rats, etc. and as such may be incorporated in a conventional dosage form, such as a tablet, capsule, elixir, injectable or the like along with the necessary carrier material, excipient, lubricant, buffer or the like. Oral dosage forms are preferred, although parenteral forms are quite satisfactory as well. Single or divided doses of from about 1 to about 320 mg, preferably from about 60 to about 240 mg one to four times daily, optimally in a single dose, may be administered in dosage forms as described above.

### Example 1

A nadolol formulation suitable for oral administration in the prevention of migraine headaches is set out below.

| Ingredient | mg/tablet |
|---|---|
| Nadolol | 40 |
| Magnesium stearate | 1 |
| Microcrystalline cellulose | 72 |

The nadolol is blended with the microcrystalline cellulose in a Hobart-type mixer for 5 minutes. Thereafter, the magnesium stearate is added with mixing for 2-3 minutes. The final mix is compressed in a Strokes D3 tablet press to form a 40 mg tablet which is used for preventing migraine headaches.

## Claims

1. The use of nadolol or an ester thereof or a pharmaceutically acceptable acid-addition salt for the preparation of drugs having activity for inhibiting the onset of migraine headaches.

2. The use as defined in claim 1 wherein said ester is a mono-, di- or tri-ester.

3. The use as defined in claim 2 wherein said ester is a monoacetate, diacetate or triacetate.

4. The use as defined in claim 1 wherein said nadolol or ester thereof is administered in a dosage of from about 1 to about 320 mg.

5. The use as defined in claim 4 wherein said nadolol or ester thereof is administered in a dosage of from about 60 to about 240 mg in a single dose.

6. The use as defined in claim 1 wherein said nadolol or ester thereof is in the form of a pharmaceutically acceptable acid-addition salt thereof.